Europäisches Patentamt

European Patent Office

Office européen des brevets

(11) Veröffentlichungsnummer: **0 343 463**
**A2**

## (12) EUROPÄISCHE PATENTANMELDUNG

(21) Anmeldenummer: 89108656.3

(22) Anmeldetag: 13.05.89

(51) Int. Cl.4: **C11D 1/72** , **C11D 17/00** ,
**A61K 7/08**

(30) Priorität: 21.05.88 DE 3817415

(43) Veröffentlichungstag der Anmeldung:
29.11.89 Patentblatt 89/48

(84) Benannte Vertragsstaaten:
**AT BE CH DE ES FR GB IT LI NL SE**

(71) Anmelder: **Henkel Kommanditgesellschaft auf Aktien**
**Postfach 1100 Henkelstrasse 67**
**D-4000 Düsseldorf 1(DE)**

(72) Erfinder: **Tesmann, Holger, Dr.**
**Vennstrasse 61**
**D-4000 Düsseldorf 12(DE)**
Erfinder: **Hensen, Hermann, Dr.**
**Rathmacherweg 13**
**D-5657 Haan(DE)**
Erfinder: **Hochschon, Wolfgang**
**Ellerstrasse 46**
**D-4010 Hilden(DE)**
Erfinder: **Ploog, Uwe, Dr.**
**Haydnweg 6**
**D-5657 Haan(DE)**
Erfinder: **Ansgar, Behler, Dr.**
**Siegfriedstrasse 80**
**D-4250 Bottrop(DE)**

(54) **Verdickte wässrige Tensidlösungen.**

(57) Anlagerungsprodukte von Ethylenoxid und/oder Propylenoxid an gesättigte und/oder ungesättigte Fettalkohole mit 8 bis 22 Kohlenstoffatomen mit mittleren Alkoxylierungsgraden n von 1 bis 10, insbesondere von 1 bis 6, sind als Verdickungsmittel für wäßrige Tensidlösungen besonders gut geeignet, wenn die Anlagerungsprodukte eine, gegenüber den durch übliche Herstellungsverfahren enthaltenen Produkten, eingeengte Homologenverteilung aufweisen.

EP 0 343 463 A2

EP 0 343 463 A2

## Verdickte wäßrige Tensidlösungen

Die Erfindung betrifft wäßrige Tensidlösungen mit speziellen Verdickungsmitteln.

Wäßrige Tensidlösungen, insbesondere solche, die im Bereich der Körperpflege als Haarshampoos, Schaumbäder, Duschbäder, Handwaschpasten und dergleichen zum Einsatz kommen, enthalten als Tensidkomponenten zumeist Aniontenside, wie beispielsweise Alkylethersulfate. Um diese klaren oder dispersen Systeme zu stabilisieren und ihre Handhabbarkeit zu verbessern, werden diesen Tensidlösungen üblicherweise Verdickungsmittel zugesetzt.

Dem Fachmann sind bereits eine Vielzahl von anorganischen und organischen Verbindungen bekannt, die zur Erhöhung der Viskosität aniontensidhaltiger wäßriger Lösungen eingesetzt werden.

Als anorganische Verdickungsmittel können eine Reihe wasserlöslicher Salze, wie beispielsweise das Kochsalz eingesetzt werden. Organische Verdickungsmittel sind u. a. Fettsäurealkanolamide, wie Kokosfettsäuremonoethanolamid, Laurinsäuremonoethanolamid, Ölsäurediethanolamid und Kokosfettsäurediethanolamid, Polyethylenglykoldifettsäureester sowie eine Reihe wasserlöslicher Polymere.

In den meisten Fällen ist es nicht oder nur unter Einsatz großer Konzentrationen möglich, allein durch Verwendung anorganischer Salze die gewünschte Viskosität in der Tensidlösung aufzubauen. Man ist daher in der Regel gezwungen, zusätzlich zu den anorganischen Salzen organische Verdickungsmittel einzusetzen, die mit einer Reihe von Nachteilen behaftet sind. So weisen die mit Polyethylenglykolfettsäurediestern verdickten Lösungen oft eine unzureichende Viskositätsstabilität bei Lagerung auf, während wasserlösliche Polymere sich oft nur schwer auflösen und ein unerwünschtes, schleimiges Fließverhalten mit der Neigung zum "Fädenziehen" bewirken. Fettsäurealkanolamide sind in kosmetischen Zubereitungen zunehmend unerwünscht, da ein geringer, herstellungsbedingter Gehalt an freien Alkanolaminen Anlaß zur Nitrosaminbildung sein kann.

In der deutschen Patentanmeldung P 37 30 179.9 wurde daher vorgeschlagen, zur Verdickung von Tensidlösungen Anlagerungsprodukte von Ethylenoxid und/oder Propylenoxid an ungesättigte Fettalkohole zu verwenden. Diese Produkte weisen die oben genannten Nachteile nicht mehr auf; es besteht jedoch das Bedürfnis nach Verdickungsmitteln mit vergleichbaren Eigenschaften bei erhöhter verdickender Wirkung. Dies würde bei gegebenem Gehalt an Verdickungsmitteln die Herstellung von Systemen mit höherer Viskosität ebenso ermöglichen wie die Verringerung des Gehaltes an Verdickungsmitteln bei vorgegebener Viskosität.

Es wurde nun überraschenderweise gefunden, daß die genannte Aufgabe gelöst werden kann, indem als Verdickungsmittel Anlagerungsprodukte von Ethylenoxid und/oder Propylenoxid an gesättigte und/oder ungesättigte Fettalkohole mit 8-22 Kohlenstoffatomen mit mittleren Alkoxylierungsgraden n von 1 bis 10, insbesondere von 1 bis 6, verwendet werden, die eine spezielle Homologenverteilung (Oligomerenverteilung) aufweisen.

Es ist dem Fachmann bekannt, daß bei Alkoxylierungsreaktionen wie beispielsweise der Anlagerung von n mol Ethylenoxid an 1 mol Fettalkohol nach den bekannten Ethoxylierungsverfahren kein einheitliches Addukt, sondern ein Gemisch aus Restmengen freien Fettalkohols und einer Reihe homologer (oligomerer) Anlagerungsprodukte von 1, 2, 3, ... n, n+1, n+2 ...usw. Molekülen Ethylenoxid je Molekül Fettalkohol erhalten wird. Der mittlere Ethoxylierungsgrad (n) wird dabei definiert durch die Ausgangsmengen an Fettalkohol und Ethylenoxid. Die Verteilungskurve des Homologengemisches weist in der Regel ein Maximum im Bereich zwischen n-3 und n+3 auf. Nähere Informationen zu diesen Punkten können beispielsweise der Zeitschrift Soap/Cosmetics/Chemical Specialities, Heft Januar 1988, S. 34, entnommen werden.

Gegenstand der Erfindung sind somit verdickte, wäßrige Tensidlösungen, enthaltend wasserlösliche, ionische Tenside sowie nichtionische Tenside als Verdickungsmittel, dadurch gekennzeichnet, daß als nichtionische Tenside Anlagerungsprodukte von Ethylenoxid und/oder Propylenoxid an gesättigte und/oder ungesättigte Fettalkohole mit 8-22 Kohlenstoffatomen mit mittleren Alkoxylierungsgraden n von 1 bis 10, insbesondere von 1 bis 6, verwendet werden, wobei die Anlagerungsprodukte eine eingeengte Homologenverteilung der Art aufweisen, daß sie signifikant weniger Homologe mit mehr als n+3 mol Ethylenoxid und/oder Propylenoxid enthalten als solche Anlagerungsprodukte, die bei Umsetzung von Fettalkoholen mit Ethylenoxid und/oder Propylenoxid unter Verwendung von etwa 0,1 bis 1 Gew.-% an Alkalimetallalkoholat, Alkalimetallhydroxid oder Alkalimetall als Katalysator bei Temperaturen von 120 °C bis 220 °C und Drucken bis 5 bar erhalten werden. Der Begriff "signifikant weniger" ist dabei so zu verstehen, daß der Gehalt an den genannten Homologen um einen Betrag reduziert ist, der erkennbar außerhalb der Meßunsicherheit der verwendeten analytischen Verfahren liegt, d. h. in der Regel größer als 5 % des Gehaltes an diesen Homologen ist.

2

Als bevorzugte, erfindungsgemäße Verdickungsmittel haben sich solche Anlagerungsprodukte erwiesen, deren Gehalt an Homologen mit mehr als n + 3 mol Ethylenoxid und/oder Propylenoxid um mehr als 10 %, insbesondere um mehr als 30 % reduziert ist im Vergleich zu den nach dem obengenannten Verfahren hergestellten Produkten. Anlagerungsprodukte, bei denen der Gehalt an den genannten Homologen um mehr als 50 % reduziert ist, zeigen besonders gute Verdickungswirkungen. Wenngleich es prinzipiell möglich ist, Anlagerungsprodukte herzustellen und erfindungsgemäß einzusetzen, die fast vollständig frei von Homologen mit mehr als n + 3 mol Ethylenoxid und/oder Propylenoxid sind, arbeitet man in der Praxis mit Produkten, deren Gehalt an den genannten Homologen vorzugsweise um maximal 90 %, insbesondere um maximal 75 % reduziert ist. Somit ist bei den erfindungsgemäßen Verdickungsmitteln der Gehalt an Homologen mit mehr als n + 3 mol Ethylenoxid und/oder Propylenoxid bevorzugt um 30 bis 90 %, insbesondere um 50 bis 75 % im Vergleich zu den nach dem obengenannten Verfahren hergestellten Produkten reduziert.

Bei den erfindungsgemäßen Alkoxylierungsprodukten kann es sich um Anlagerungsprodukte von Ethylenoxid, Propylenoxid oder von Gemischen aus Ethylenoxid und Propylenoxid an Fettalkohole handeln. In der Regel zeigen solche Produkte die besseren Verdickungseigenschaften, bei denen nur Ethylenoxid oder eine Ethylenoxid/Propylenoxid-Mischung, die überwiegend aus Ethylenoxid besteht, an den Fettalkohol angelagert wird. Der Einsatz dieser Produkte ist daher bevorzugt. Besonders bevorzugt ist die Verwendung von reinen Ethoxylierungsprodukten.

Innerhalb der Anlagerungsprodukte von Ethylenoxid an Fettalkohole zeigen wiederum solche Produkte die besten Verdickungseigenschaften, die bei 20 °C nicht oder nur in geringem Maße in Wasser löslich sind; gleichwohl können diese Produkte durch das zu verdickende Tensid solubilisiert werden.

Die erfindungsgemäßen Verdickungsmittel lassen sich nach unterschiedlichen Verfahren herstellen.

So ist es zum einen möglich, zunächst nach bekannten Verfahren, beispielsweise durch Umsetzung der Fettalkohole mit Ethylenoxid und/oder Propylenoxid unter der katalytischen Wirkung von Alkalimetallalkoholaten, wie Natriummethylat, oder Alkalimetallhydroxiden Anlagerungsprodukte mit weiter Homologenverteilung herzustellen. Durch physikalische und/oder chemische Trennverfahren können dann die entstandenen höheren Homologen soweit abgetrennt werden, bis Produkte mit der gewünschten Homologenverteilung vorliegen. Ein einfaches und vergleichsweise kostengünstiges Trennverfahren stellt die Destillation dar.

Es ist jedoch bevorzugt, durch entsprechende Wahl des Katalysatorsystems bereits bei der Herstellung der Anlagerungsprodukte dafür zu sorgen, daß das Produktgemisch eine eingeengte Homologenverteilung aufweist. So ist beispielsweise aus den Europäischen Patentschriften 6 105, 26 544, 26 546, 82 569, 92 256 und 115 083 bekannt, daß durch Verwendung von Erdalkalimetalloxiden, -hydroxiden oder -alkoxiden, gegebenenfalls in Kombination mit Co-Katalysatoren und Promotern, als Katalysatoren Produkte mit eingeengten Homologenverteilungen erhalten werden können. In der Deutschen Patentanmeldung P 37 06 047.3 werden Erdalkalimetallsalze vicinal hydroxy, alkoxy-substituierter Fettsäuren beschrieben, deren Verwendung als Katalysatoren bei Alkoxylierungsreaktionen zu Produkten mit eingeengter Homologenverteilung führt. Der Einsatz von Erdalkalimetallsalzen von Ethercarbonsäuren als Katalysatoren führt ebenfalls, wie aus den Deutschen Patentanmeldungen P 37 19 968.4 und P 38 02 044.0 bekannt, zu Produkten mit eingeengter Homologenverteilung. Nach den Lehren weiterer deutscher Patentanmeldungen führt auch die Verwendung folgender Alkoxylierungskatalysatoren zu stark eingeengten Homologenverteilungen: Ester von Titan- und/oder Zirkonsäure mit Monoalkanolen in Kombination mit Schwefelsäure und/oder Alkansulfonsäuren und/oder Hydroxyalkylsulfonsäuren (P 38 14 849.8), Erdalkalimetallsalze von Polycarbonsäuremonoestern (P 38 12 168.9) sowie Hydrotalcit (P 38 13 910.3).

Gleichwohl also die Herstellung von Alkoxylierungsprodukten mit eingeengter Homologenverteilung in einer Reihe von Druckschriften beschrieben wird, lassen sich diesen jedoch keine Hinweise auf die ausgezeichnete Verdickungswirkung dieser Produkte entnehmen.

In vielen Fällen weisen die mit den genannten Katalysatoren erhaltenen Produkte bereits solche eingeengten Homologenverteilungen auf, daß sie direkt als Verdickungsmittel in den erfindungsgemäßen Tensidlösungen eingesetzt werden können. Es kann jedoch in einzelnen Fällen notwendig sein, durch Einsatz der oben geschilderten Trennoperationen den Anteil der Alkoxylierungsprodukte mit mehr als n + 3 mol Ethylenoxid und/oder Propylenoxid noch weiter zu verringern, bis die Produkte den erfindungsgemäßen Anforderungen entsprechen.

Als besonders geeignete Verdickungsmittel haben sich Alkoxylierungsprodukte mit mittleren Alkoxylierungsgraden von 2 bis 5, insbesondere von 2 bis 4, erwiesen. Die Verwendung dieser Verbindungen in den erfindungsgemäßen Tensidlösungen ist daher bevorzugt.

Basis der Alkoxylierungsprodukte sind gesättigte und/oder olefinisch ungesättigte Fettalkohole mit 8 bis 22 Kohlenstoffatomen. Diese Fettalkohole können sowohl verzweigte und auch unverzweigte Kohlenstoffketten aufweisen. Die unverzweigten Fettalkohole werden jedoch aufgrund der besseren Verdickungseigen-

3

schaften ihrer Alkoxylierungsprodukte bevorzugt eingesetzt. Es ist möglich, reine Fettalkohole oder Gemische verschiedener Fettalkohole einzusetzen. In der Regel wird man Fettalkohole einsetzen, die aus nativen Rohstoffen, wie natürlichen Fetten und Ölen, gewonnen werden. In diesem Fall werden dann Fettalkoholgemische mit einer, von der Herkunft abhängigen, Verteilung der Kohlenstoffkettenlängen erhalten. Durch Auswahl geeigneter Katalysatoren kann gewünschtenfalls sichergestellt werden, daß bei der reduzierenden Umwandlung der Carbonsäuregruppierung zur Alkoholgruppe in den Kohlenstoffketten vorliegende ethylenische Doppelbindungen nicht oder nicht wesentlich hydriert werden. Es ist bevorzugt, Fettalkoholgemische zu verwenden, die überwiegend Fettalkohole mit Kettenlängen von $C_{12}$ bis $C_{18}$, insbesondere von $C_{16}$ bis $C_{18}$, enthalten. Solche Gemische lassen sich beispielsweise aus Kokosöl, Rapsöl oder Rindertalg gewinnen und werden von der Anmelderin unter Handelsnamen wie "Ocenol" und "Lorol" vertrieben.

Die erfindungsgemäßen verdickten wäßrigen Tensidlösungen enthalten üblicherweise 3 bis 30 Gew.-% ionischer Tenside, 0,5 bis 5 Gew.-% wasserlöslicher organischer Verdickungsmittel und 0 bis 10 Gew.-% wasserlöslicher anorganischer und/oder organischer Elektrolytsalze.

Als wasserlösliche ionische Tenside können anionische, zwitterionische und kationische Tenside enthalten sein. Geeignete ionische Tenside zeichnen sich durch eine lipophile, bevorzugt lineare Alkyl- oder Alkenylgruppe mit 8 - 18 C-Atomen und eine, bevorzugt endständig daran gebundene, in Wasser dissoziierende ionische Gruppe aus. Die anionische Gruppe kann z. B. eine Sulfat-($-OSO_3^-$), Sulfonat-($-SO_3^-$), Phosphat-($-O-PO_3^{--}$), oder Carboxylat-($-COO^-$)-Gruppe sein, die kationische Gruppe kann z. B. eine quartäre Ammoniumgruppe (z. B.$-N^+(CH_3)_3$) sein, die zwitterionischen Gruppen können z. B. $-N^+(CH_3)_2-CH_2-COO^-$ oder $-N^+(CH_3)_2-CH_2-\underset{OH}{C}H-CH_2-SO_3^-$ sein.

Geeignete und bevorzugte anionische Tenside sind z. B. Alkylsulfate, Alkylpolyglycolethersulfate, Alkansulfonate, Sulfobernsteinsäuremonoalkylester- und -monopolyethoxyalkylester, Monoalkylphosphate, Eiweißfettsäurekondensationsprodukte, Acylisethionate, Acyltauride, Seifen, Alkylethercarbonsäuren und Acylsarcoside. Geeignete kationische Tenside sind z. B. Alkyl-trimethylammonium-halogenide, Dialkyl-dimethylammonium-halogenide, Alkyl-dimethyl-benzylammonium-halogenide, Alkyl-pyridinium-halogenide und Alkylimidazolinium-halogenide. Geeignete zwitterionische Tenside sind z. B. N-Alkyl-N,N-dimethylglycin oder N-Acylaminopropyl-N,N-dimethylglycin, ferner Alkylaminocarbonsäuren, wie sie bei der Umsetzung von Kondensationsprodukten von Fettsäuren mit Aminoethylethanolaminen ("Imidazolinen") mit Natriumchloracetat etc. erhalten werden. Als ionische Tenside können auch Gemische der genannten anionischen oder Gemische aus anionischen oder kationischen und zwitterionischen Tensiden verwendet werden.

Von besonders hohem anwendungstechnischen Interesse ist die Viskositätserhöhung von wässrigen Lösungen der genannten Art mit vergleichsweise geringem Gehalt an ionischen Tensiden. In diesen Fällen verbessert der Zusatz der erfindungsgemäß ausgewählten Verdickungsmittel in synergistischer Weise die Verdickbarkeit mit anorganischen Elektrolytsalzen.

Als anorganische Elektrolytsalze eignen sich alle wasserlöslichen Alkali-, Ammonium- und Erdalkalisalze, z. B. die Fluoride, Chloride, Bromide, Sulfate, Phosphate, Nitrate, soweit sie bei 20 °C in einer Menge von wenigstens 1 Gew.-% in Wasser löslich sind. Bevorzugt werden die Chloride oder Sulfate eines Alkalimetalls, des Ammoniums oder des Magnesiums verwendet. Besonders bevorzugt sind Natriumchlorid und Magnesiumchlorid.

Als organische Elektrolytsalze eignen sich besonders alle wasserlöslichen Alkali-, Ammonium- und Erdalkalisalze von Mono-, Di- und Tricarbonsäuren. Bevorzugt sind dabei solche Carbonsäuren, die eine Molmasse kleiner 200 g/mol aufweisen, beispielsweise Bernsteinsäure, Weinsäure und Glutarsäure. Auch Mischungen dieser Salze können erfindungsgemäß eingesetzt werden.

Die erfindungsgemäßen wäßrigen Zubereitungen können darüber hinaus weitere Komponenten enthalten, die sie für den jeweiligen Anwendungszweck geeignet machen. In Betracht kommen beispielsweise nichtionogene Tenside, bevorzugt in kleineren Mengen bis höchstens etwa 50 Gew.-% der vorliegenden ionischen Tenside. In untergeordneten Mengen können schließlich Duftstoffe, Farbstoffe, Trübungs-und Perlglanzmittel, antimikrobielle Stoffe, Konservierungsmittel, hautkosmetische Wirkstoffe, Pflanzenextrakte, Proteinhydrolysate, Puffersubstanzen, Komplexbildner und andere bekannte Hilfs- und Zusatzstoffe enthalten sein, wie sie in Shampoos, Badezusätzen, Duschbadpräparaten, flüssigen Seifen, flüssigen Hautreinigungsmitteln, flüssigen Haarspülmitteln, aber auch in flüssigen Wasch-und Geschirrspülmitteln sowie flüssigen Haushaltsreinigungsmitteln auf Basis von ionischen Tensiden üblich sind.

Die erfindungsgemäße Lehre wird in den folgenden Beispielen verdeutlicht, ohne daß sie darauf beschränkt ist.

## Beispiele

## I. Eingesetzte Fettalkohole

Den in den Beispielen verwendeten Alkoxylierungsprodukten liegen die folgenden Fettalkohole zugrunde:

### 1) Lorol Spezial

Unter der Bezeichnung "LOROL SPEZIAL" wird von der Anmelderin eine Mischung von Fettalkoholen vertrieben, die folgende C-Ketten-Verteilung aufweist:

| C-Kette | Spezifikation | typischer Gehalt |
|---------|---------------|------------------|
|         | (Gew.-%)      |                  |
| C-10    | 0 - 2         | ca. 1            |
| C-12    | 70 - 75       | ca. 72           |
| C-14    | 25 - 30       | ca. 26           |
| C-16    | 0 - 2         | ca. 1            |

### 2) "Rübocenol"

Die C-Kettenverteilung dieses aus erucasäurearmem Rapsöl gewonnenen ungesättigten Fettalkohols entspricht etwa der folgenden Zusammensetzung:

| C-Kette | Gew.-% |
|---------|--------|
| C-16 | ca. 7,0 |
| C-16$'$ | ca. 2,0 (Palmitoleylalkohol) |
| C-18 | ca. 3,0 |
| C-18$'$ | ca. 70,0 (Oleylalkohol) |
| C-18$''$ | ca. 8,0 (Linoleylalkohol) |
| C-18$'''$ | ca. 7,0 (Linolenylalkohol) |
| C-20 | ca. 3,0 |

### 3) HD-Ocenol 50/55

Die C-Kettenverteilung des von der Anmelderin unter dieser Bezeichnung vertriebenen Produktes, einer Mischung von im wesentlichen ungesättigten Fettalkoholen, ist wie folgt:

| C-Kette | Spezifikation | typischer Gehalt |
|---------|---------------|------------------|
|         | (Gew.-%)      |                  |
| C-12    | 0 - 2         | ca. 1            |
| C-14    | 2 - 7         | ca. 5            |
| C-16    | 25 - 35       | ca. 30           |
| C-18    |               | ca. 20           |
| C-18'   | 55 - 75       | ca. 41           |
| C-18''  |               | ca. 1            |
| C-18''' |               | ca. 1            |
| C-20    | 0 - 2         | ca. 1            |

In diesen Tabellen ist die jeweilige Anzahl der vorliegenden ethylenischen Doppelbindungen durch die Anzahl der hochgesetzten '-Zeichen dargestellt.

## II. Alkoxylierungsprodukte

Aus den genannten Fettalkoholen wurden nach folgender Vorschrift ethoxylierte Produkte hergestellt:

Der zu ethoxylierende Alkohol wurde mit dem Katalysatorsystem (ca. 0,1 bis 1 Gew.-%, bezogen auf das Endprodukt) versetzt und in einen geeigneten Autoklaven überführt. Anschließend wurde mit Stickstoff gespült und ca. 30 Minuten lang bei einer Temperatur von 100 °C evakuiert. Sodann wurde die Temperatur auf 180 °C gesteigert und die erforderliche Menge Ethylenoxid (EO) bei einem Druck von max. 5 bar aufgedrückt. Nach Beendigung der Reaktion ließ man noch 30 Minuten nachreagieren und zog den Rest des Ethylenoxids bei 100 °C und einem Druck von 14 mbar ab.

Die für die Beispiele und Vergleichsbeispiele hergestellten Verdickungsmittel sind in Tabelle 1 aufgeführt:

Tabelle 1

| Verdickungsmittel | Fettalkohol | n | Katalysator |
|-------------------|-------------|-----|-------------|
| B 1 | Lorol Spezial | 3 | Calciumsalz einer Alkoxyhydroxyfettsäure[1] |
| V 1 | Lorol Spezial | 3 | Natriummethylat |
| B 2a | Rübocenol | 3,5 | Calciumsalicylat |
| B 2b | Rübocenol | 3,5 | Hydrotalcit[2] |
| V 2 | Rübocenol | 3,5 | Natriummethylat |
| B 3 | HD-Ocenol 50/55 | 3 | Erdalkalisalz einer Ethercarbonsäure[3] |
| V 3 | HD-Ocenol 50/55 | 3 | Natriummethylat |
| B 4 | Lorol Spezial | 2,5 | Hydrotalcit[2] |
| V4 | Lorol Spezial | 2,5 | Natriummethylat |

[1] nach deutscher Patentanmeldung P 37 06 047.3
[2] nach Deutscher Patentanmeldung P 38 13 910.3
[3] nach Deutscher Patentanmeldung P 37 19 968.4

Die Homologen-Verteilung der erhaltenen Produkte ist in Tabelle 2 aufgeführt. Die Daten wurden wie folgt erhalten:

Die endständigen Hydroxylgruppen der Alkoxylierungsprodukte wurden zur Erhöhung der Flüchtigkeit der Verbindungen mit Acetylchlorid nach bekannten Methoden zu den Acetaten umgesetzt. Zur gaschromatographischen Trennung der Homologen wurden 1 µl einer 1%igen Lösung der Acetate in Isooctan mittels einer 2 m langen Glassäule getrennt, deren stationäre Phase aus 3 % SE 30 (Fa. Supelco) auf Chromosorb GAWDMCS (Fa. Waters) bestand. Als Trägergas wurde Stickstoff mit einer Durchflußrate von 25 ml/min verwendet. Die Temperatur der Kolonne wurde während der Trennung von 120 °C auf 300 °C mit einer Rate von 4 °C/min erhöht. Zur Erfassung der getrennten Produkte diente ein Flammenionisationsdetektor (Packard 438).

## Tabelle 2

| Substanz | Flächenprozent | | | | |
| --- | --- | --- | --- | --- | --- |
| | B 1 | V 1 | B 2a | B 2b | V 2 |
| Freier Fettalkohol | 14,0 | 21,5 | 10,8 | 5,6 | 18,1 |
| Fettalkohol +  1 EO | 10,5 | 13,7 | 7,9 | 7,0 | 10,7 |
| Fettalkohol +  2 EO | 14,2 | 12,4 | 11,1 | 10,0 | 10,4 |
| Fettalkohol +  3 EO | 16,5 | 10,8 | 14,6 | 20,3 | 10,2 |
| Fettalkohol +  4 EO | 15,7 | 9,1 | 13,3 | 19,7 | 9,0 |
| Fettalkohol +  5 EO | 12,0 | 7,0 | 10,0 | 13,6 | 7,2 |
| Fettalkohol +  6 EO | 7,4 | 5,8 | 6,0 | 7,4 | 6,2 |
| Fettalkohol +  7 EO | 3,8 | 4,7 | 3,1 | 3,1 | 5,1 |
| Fettalkohol +  8 EO | 1,7 | 3,6 | 1,2 | 1,0 | 4,1 |
| Fettalkohol +  9 EO | 0,8 | 2,7 | 0,4 | 0,5 | 3,0 |
| Fettalkohol + 10 EO | 0,2 | 1,9 | 0,2 | 0,3 | 1,8 |
| Fettalkohol + 11 EO | - | 1,2 | 0,1 | 0,2 | 1,0 |
| Fettalkohol + 12 EO | - | 0,8 | - | 0,1 | 0,5 |
| Fettalkohol + 13 EO | - | 0,4 | - | - | 0,1 |
| Fettalkohol + 14 EO | - | 0,2 | - | - | - |

7

Tabelle 2    Fortsetzung

| Substanz | Flächenprozent | | | |
|---|---|---|---|---|
| | B 3 | V 3 | B4 | V4 |
| Freier Fettalkohol | 17,0 | 18,6 | 12,1 | 26,2 |
| Fettalkohol +  1 EO | 11,3 | 11,3 | 15,8 | 16,9 |
| Fettalkohol +  2 EO | 14,1 | 10,6 | 22,9 | 14,9 |
| Fettalkohol +  3 EO | 15,7 | 9,6 | 24,1 | 12,0 |
| Fettalkohol +  4 EO | 14,2 | 7,9 | 15,2 | 9,3 |
| Fettalkohol +  5 EO | 5,7 | 6,4 | 6,2 | 6,6 |
| Fettalkohol +  6 EO | 3,5 | 5,6 | 1,9 | 4,9 |
| Fettalkohol +  7 EO | 1,7 | 4,4 | 0,6 | 3,4 |
| Fettalkohol +  8 EO | 1,4 | 3,4 | 0,2 | 2,3 |
| Fettalkohol +  9 EO | 0,5 | 2,5 | 0,1 | 1,3 |
| Fettalkohol + 10 EO | 0,4 | 1,7 | 0,2 | |
| Fettalkohol + 11 EO | 0,3 | 1,3 | 0,1 | |
| Fettalkohol + 12 EO | 0,2 | 0,9 | 0,1 | |
| Fettalkohol + 13 EO | 0,4 | 0,7 | | |
| Fettalkohol + 14 EO | 0,2 | 0,2 | | |

## III. Anwendungstechnische Untersuchungen der Tensidlösungen

Es wurden die sich jeweils einstellenden Viskositätswerte wäßriger Lösungen bestimmt, die der folgenden Grundzusammensetzung entsprechen:
10 Gew.-% Ethersulfat (Tensid)
3 Gew.-% Verdickungsmittel
x Gew.-% NaCl

8

Als Ethersulfat (Tensid) wurde das von der Anmelderin unter dem Handelsnamen "Texapon NSO" vertriebene Produkt (Fettalkohol $C_{12/14}$(70:30)polyglykolether-(2 EO)-sulfat, Na-Salz, 28 %ige Lösung in Wasser) eingesetzt.

Die mit Hilfe eines Höppler-Kugelfallviskosimeters bei 20 °C bestimmten Viskositätswerte der Tensidlösungen mit dem Verdickungsmittel mit eingeengter Homologenverteilung (B1-B3) sind in Tabelle 3 aufgeführt. Ein Vergleich mit den Werten, die sich bei Verwendung der Verdickungsmittels mit breiter Homologenverteilung (V1-V3) ergeben, zeigt die Vorteile der erfindungsgemäßen Verdikkungsmittel.

Tabelle 3

| Verdickungsmittel | Gehalt an NaCl x (Gew.-%) | Viskosität (mPas) | Trübungspunkt (°C) | Klarpunkt (°C) |
|---|---|---|---|---|
| B 1 | 2,0 | 25000 | 0 | 14 |
| V 1 | 2,0 | 16000 | 7 | 19 |
| B 2a | 2,5 | 43000 | 10 | 25 |
| B 2b | 2,75 | 62000 | 11 | 23 |
| V 2 | 3,0 | 37000 | 7 | 26,5 |
| B 3 | 2,5 | 60000 | 20 | 25 |
| V 3 | 2,5 | 36000 | 20 | 26,5 |
| B4 | 1,5 | 41200 | 6 | 23 |
| V4 | 1,5 | 4900 | | |

## Ansprüche

1. Verdickte, wäßrige Tensidlösungen, enthaltend wasserlösliche, ionische Tenside sowie nichtionische Tenside als Verdickungsmittel, dadurch gekennzeichnet, daß als nichtionische Tenside Anlagerungsprodukte von Ethylenoxid und/oder Propylenoxid an gesättigte und/oder ungesättigte Fettalkohole mit 8-22 Kohlenstoffatomen mit mittleren Alkoxylierungsgraden n von 1 bis 10, insbesondere von 1 bis 6, verwendet werden, wobei die Anlagerungsprodukte eine eingeengte Homologenverteilung der Art aufweisen, daß sie signifikant weniger Homologe mit mehr als n+3 mol Ethylenoxid und/oder Propylenoxid enthalten als solche Anlagerungsprodukte, die bei Umsetzung von Fettalkoholen mit Ethylenoxid und/oder Propylenoxid unter Verwendung von etwa 0,1 bis 1 Gew.-% an Alkalimetallalkoholat, Alkalimetallhydroxid oder Alkalimetall als Katalysator bei Temperaturen von 120 °C bis 220 °C und Drucken bis 5 bar erhalten werden.

2. Wäßrige Tensidlösungen nach Anspruch 1, dadurch gekennzeichnet, daß Anlagerungsprodukte von Ethylenoxid verwendet werden.

3. Wäßrige Tensidlösungen nach einem der Ansprüche 1 oder 2, dadurch gekennzeichnet, daß Anlagerungsprodukte verwendet werden, deren Gehalt an Homologen mit mehr als n+3 mol Ethylenoxid und/oder Propylenoxid um mehr als 10 %, insbesondere um mehr als 30 %, reduziert ist.

4. Wäßrige Tensidlösungen nach einem der Ansprüche 1 bis 3, dadurch gekennzeichnet, daß die Anlagerungsprodukte einen mittleren Alkoxylierungsgrad n von 2 bis 5, insbesondere von 2 bis 4 aufweisen.

5. Wäßrige Tensidlösungen nach einem der Ansprüche 1 bis 4, dadurch gekennzeichnet, daß Fettalkohole mit einem überwiegenden Anteil der Kettenlänge $C_{12}$-$C_{18}$, insbesondere $C_{16}$-$C_{18}$, verwendet werden.

6. Wäßrige Tensidlösungen nach einem der Ansprüche 1 bis 5, dadurch gekennzeichnet. daß sie 3 bis 30 Gew.-% ionischer Tenside, 0,5 bis 5 Gew.-% wasserlöslicher organischer Verdickungsmittel und 0 bis 10 Gew.-% wasserlöslicher anorganischer und/oder organischer Elektrolytsalze enthalten.